Europäisches Patentamt

European Patent Office

Office européen des brevets

(19)

(11) Publication number: **0 261 754 B1**

(12) **EUROPEAN PATENT SPECIFICATION**

(45) Date of publication of patent specification: **11.09.91**

(51) Int. Cl.5: **A61K 7/50, C11D 17/08**

(21) Application number: **87302304.8**

(22) Date of filing: **18.03.87**

(54) Encapsulated products.

(30) Priority: **09.07.86 GB 8616681**

(43) Date of publication of application:
**30.03.88 Bulletin 88/13**

(45) Publication of the grant of the patent:
**11.09.91 Bulletin 91/37**

(84) Designated Contracting States:
**AT BE CH DE ES FR GB GR IT LI LU NL SE**

(56) References cited:
**DE-B- 1 069 334**
**GB-A- 2 118 961**
**US-A- 3 705 102**
**US-A- 4 597 885**

(73) Proprietor: **R.P. Scherer Limited**
**Frankland Road Blagrove**
**Swindon Wiltshire SN5 8YS(GB)**

(72) Inventor: **Tanner, Keith Edward**
**Flat 13, Stratford Close**
**Toothill, Swindon(GB)**

(74) Representative: **Lamb, John Baxter et al**
**MARKS & CLERK 57/60 Lincoln's Inn Fields**
**London WC2A 3LS(GB)**

## Description

This invention is concerned with improvements in and relating to surfactant compositions and, more particularly, is concerned with encapsulated surfactant products suitable for use as foam bath compositions, that is compositions for addition to the water of a bath to produce a foam thereon.

Foam bath surfactant compositions are well known and widely available. Whilst they are often put up in dosage unit form, i.e. in the form of sachets, it would be useful to put them up in a dosage unit which could be simply added to the water of a bath, i.e. in a dosage unit having a water-soluble envelope.

According to the present invention there is provided a substantially anhydrous composition encapsulated in a soft elastic gelatin capsule and comprising (i) from 40 to 60% by weight, preferably about 50% by weight, of one or more amine salts of long chain alkyl ether sulphates (suitably alkyl ether sulphates containing a $C_8$ - $C_{20}$ alkyl group, such as a lauryl group, and containing, on average, from 2 to 30 moles of ethylene oxide per molecule); (ii) from 25% to 35% by weight, preferably about 30% by weight, of one or more liquid polyethylene glycols such as PEG 400 or PEG 600); and from 2.5 to 7.5% by weight of glycerine.

In addition to the above components the compositions of the invention may also contain other ingredients, especially perfumes (which are suitably present in amounts of from 3 to 5% by weight) and one or more foam boosters which are suitably present in amounts of from 5 to 15% by weight.

Examples of suitable foam boosters include long chain alkyl mono- or di-alkanolamides (e.g. coconut diethanolamide) and amphoteric surfactants such as alkyl amidobetaines (e.g. cocoamidopropyl betaine), imidazolines and imidazoline betaines.

The total volume of the composition encapsulated within the soft elastic gelatin capsule is suitably from 1 to 5 ml, preferably about 4 ml. The composition, the "fill" may be encapsulated in the gelatin capsule by conventional methods e.g. by means of the well known rotary drum apparatus. The gelatin shell will normally be of conventional composition, i.e. comprising gelatin, water and a plasticizer (such as glycerol or sorbitol), suitably in relative amounts of 10 to 40%.

In order that the invention may be well understood the following example is given by way of illustration only.

Soft elastic gelatine capsules having a volume of about 4 ml were made, each capsule containing the following fill:

| | |
|---|---|
| Akyposal 100 LFS (amine salts of long chain alkyl ether sulphate) | 2000 mg |
| Rewomid DL 240 (coconut diethanolamide) | 400 mg |
| Polyethylene glycol 400 BP | 1200 mg |
| Glycerine | 200 mg |
| Perfume (E 1011) | 200 mg |

Two such capsules when introduced into a bath while being filled produced a stable, pleasant foam on the surface of the bath water.

## EP 0 261 754 B1

**Claims**

1. A substantially anhydrous composition encapsulated in a soft elastic gelatin capsule and comprising (i) from 40 to 60% by weight of one or more amine salts of long chain alkyl ether sulphates; (ii) from 25 to 35% by weight of one or more liquid polyethylene glycols; and (iii) from 2.5 to 7.5% by weight of glycerin.

2. An encapsulated composition as claimed in claim 1 also containing one or more of perfumes and foam boosters.

3. An encapsulated composition as claimed in claim 2 in which the foam booster is a long chain mono- or dialkanolamide or an alkylamidobetaine.

4. An encapsulated composition as claimed in claim 2 or claim 3 containing from 5 to 15% by weight of foam booster.

5. An encapsulated composition as claimed in any one of the preceding claims having a volume of from 3 to 5 ml.

**Revendications**

1. Un composé pratiquement anhydre encapsulé dans une capsule molle et souple en gélatine, comprenant (i) entre 40 à 60% en poids d'un ou de plusieurs sels d'amines de sulfates d'alkyl-éther à chaîne longue; (ii) de 25 à 35% en poids d'un ou de plusieurs polyéthylène-glycols liquides; et (iii) de 2,5 à 7,5% en poids de glycérine.

2. Un composé encapsulé selon la revendication 1, contenant également un ou plusieurs parfums et renforçateurs de mousse.

3. Un composé encapsulé selon la revendication 2, dans lequel le renforçateur de mousse est une mono ou dialkanolamide à chaîne longue ou une alkylamidobétaïne.

4. Un composé encapsulé selon la revendication 2 ou la revendication 3, contenant de 5 à 15% en poids de renforçateur de mousse.

5. Un composé encapsulé selon l'une quelconque des revendications précédentes, ayant un volume de 3 à 5 ml.

**Patentansprüche**

1. Im wesentlichen wasserfreie Zusammensetzung, die in einer weichen elastischen Gelatinekapsel eingekapselt ist und (i) 40 bis 60 Gew.-% eines oder mehrerer Aminsalze langkettiger Alkylethersulfate; (ii) 25 bis 35 Gew.-% eines oder mehrerer flüssiger Polyethylenglykole; und (iii) 2,5 bis 7,5 Gew.-% Glyerin umfaßt.

2. Eingekapselte Zusammensetzung nach Anspruch 1, enthaltend ebenso einen oder mehrere Riechstoffe und Schaumverstärker.

3. Eingekapselte Zusammensetzung nach Anspruch 2, wobei der Schaumverstärker ein langkettiges Mono- oder Dialkanolamid oder ein Alkylamidobetain ist.

4. Eingekapselte Zusammensetzung nach Anspruch 2 oder 3, enthaltend 5 bis 15 Gew.-% Schaumverstärker.

5. Eingekapselte Zusammensetzung nach mindestens einem der vorangehenden Ansprüche mit einem Volumen von 3 bis 5 ml.